# EUROPEAN PATENT APPLICATION

(11) **EP 0 896 056 A1**
(43) Date of publication of application: **10.02.1999**
(21) Application number: 96941836.7
(22) Date of filing: 11.12.1996
(51) Int. Cl.: C12N 9/54, C12P 21/02, C12N 15/57

(54) **NOVEL THERMOLYSIN-LIKE PROTEASE AND USE THEREOF**

(30) Priority: 11.12.1995 JP 321814/95
(71) Applicant: SAGAMI CHEMICAL RESEARCH CENTER, Sagamihara-shi, Kanagawa 229 (JP); HOLLAND SWEETENER COMPANY V.O.F., NL-6212 XW Maastricht (NL)
(72) Inventor: HANZAWA, Satoshi, Ebina-shi, Kanagawa 243-04 (JP); OHE, Seigo, Yokohama-shi, Kanagawa 227 (JP); MIYAKE, Toshio, Yokohama-shi, Kanagawa 244 (JP); KIDOKORO, Shun-ichi, Sagamihara-shi, Kanagawa 228 (JP); ENDO, Kimiko, Machida-shi, Tokyo 194 (JP); WADA, Akiyoshi, Tokyo 107 (JP)
(74) Representative: Jacobs, Monique S.N.
(86) International application number: JP9603617
(87) International publication number: WO9721804

(57) **Abstract**

An object of this invention is to provide a novel thermolysin-like protease having improved stability, and it also provides a method in which this protease is used in a coupling reaction with phenylalanine methyl ester to improve efficiency of the coupling reaction. This invention relates to a novel thermolysin-like protease which is characterized in that a novel thermolysin-like protease in which the volume of an electron density cavity that exists in a thermolysin molecule having the amino acid sequence shown in SEQ ID NO:1 of the Sequence Listing and is surrounded by at least the 144th position leucine residue, the 149th position threonine residue, the 240th position alanine residue, the 270th position alanine residue and the 288th position alanine residue is reduced by filling up at least a portion of the cavity through replacement of one or more of these amino acid residues by other amino acid residues having higher hydrophobic character and being bulkier than those of the former amino acid residues, or through exertion of an influence upon the amino acid residues which surround the cavity in electron density. The invention also relates to a method for the synthesis of benzyloxycarbonyl-α-L-aspartyl-L-phenylalanine methyl ester by an enzyme reaction in which such a thermolysin-like protease is used for the coupling of N-benzyloxycarbonylaspartic acid with phenylalanine methyl ester, and to a method in which the protease is used for the coupling of N-benzyloxycarbonylaspartic acid with phenylalanine methyl ester in a water-soluble medium.

SEQUENCE LISTING

<110> Sagami Chemical Research Center
      Holland Sweetner Company V.o.f.

<120> Novel Thermolysin-like Protease and use Thereof

<130> Novel Thermolysin-like protease

<140> 96941836.7
<141> 1996-12-11

<150> 7-321814
<151> 1995-12-11

<160> 4

<170> PatentIn Ver. 2.0

<210> 1
<211> 316
<212> PRT
<213> the genus Bacillus

<220>

<400> 1
Ile Thr Gly Thr Ser Thr Val Gly Val Gly Arg Gly Val Leu Gly Asp
  1               5                  10                  15

Gln Lys Asn Ile Asn Thr Thr Tyr Ser Thr Tyr Tyr Tyr Leu Gln Asp
             20                  25                  30

Asn Thr Arg Gly Asn Gly Ile Phe Thr Tyr Asp Ala Lys Tyr Arg Thr
         35                  40                  45

Thr Leu Pro Gly Ser Leu Trp Ala Asp Ala Asp Asn Gln Phe Phe Ala
     50                  55                  60

Ser Tyr Asp Ala Pro Ala Val Asp Ala His Tyr Tyr Ala Gly Val Thr
 65                  70                  75                  80

Tyr Asp Tyr Tyr Lys Asn Val His Asn Arg Leu Ser Tyr Asp Gly Asn
                 85                  90                  95

Asn Ala Ala Ile Arg Ser Ser Val His Tyr Ser Gln Gly Tyr Asn Asn
            100                 105                 110

Ala Phe Trp Asn Gly Ser Gln Met Val Tyr Gly Asp Gly Asp Gly Gln
        115                 120                 125

Thr Phe Ile Pro Leu Ser Gly Gly Ile Asp Val Val Ala His Glu Leu
    130                 135                 140

Thr His Ala Val Thr Asp Tyr Thr Ala Gly Leu Ile Tyr Gln Asn Glu
145                 150                 155                 160

Ser Gly Ala Ile Asn Glu Ala Ile Ser Asp Ile Phe Gly Thr Leu Val
                165                 170                 175

Glu Phe Tyr Ala Asn Lys Asn Pro Asp Trp Glu Ile Gly Glu Asp Val
            180                 185                 190

Tyr Thr Pro Gly Ile Ser Gly Asp Ser Leu Arg Ser Met Ser Asp Pro
        195                 200                 205

Ala Lys Tyr Gly Asp Pro Asp His Tyr Ser Lys Arg Tyr Thr Gly Thr
    210                 215                 220

Gln Asp Asn Gly Gly Val His Ile Asn Ser Gly Ile Ile Asn Lys Ala
225                 230                 235                 240

Ala Tyr Leu Ile Ser Gln Gly Gly Thr His Tyr Gly Val Ser Val Val
                245                 250                 255

Gly Ile Gly Arg Asp Lys Leu Gly Lys Ile Phe Tyr Arg Ala Leu Thr
            260                 265                 270

Gln Tyr Leu Thr Pro Thr Ser Asn Phe Ser Gln Leu Arg Ala Ala Ala
        275                 280                 285

Val Gln Ser Ala Thr Asp Leu Tyr Gly Ser Thr Ser Gln Glu Val Ala
    290                 295                 300

Ser Val Lys Gln Ala Phe Asp Ala Val Gly Val Lys
305                 310                 315

<210> 2
<211> 23
<212> DNA
<213> Artificial Sequence

<220>
<223> Description of Artificial Sequence:
oligonucleotide (synthetic ss-DNA)

<400> 2
taccgcatgc gtgaactcat gtg                                              23

<210> 3
<211> 23
<212> DNA
<213> Artificial Sequence

<220>
<223> Description of Artificial Sequence:
      oligonucleotide (synthetic ss-DNA)

<400> 3
taccgcatgc gtgtactcat gtg                                              23

<210> 4
<211> 23
<212> DNA
<213> Artificial Sequence

<220>
<223> Description of Artificial Sequence:
      oligonucleotide (synthetic ss-DNA)

<400> 4
taccgcatgc gtsndctcat gtg                                              23

## Description

### TECHNICAL FIELD

This invention relates to an improved method for the synthesis of benzyloxycarbonyl-α-L-aspartyl-L-phenylalanine methyl ester (referred to as "Z-APM" hereinafter) by an enzyme reaction, in which a thermolysin-like protease is used in the coupling reaction of N-benzyloxycarbonylaspartic acid (referred to as "Z-Asp" hereinafter, wherein the term "Z-Asp" means "Z-L-Asp" or "Z-Asp racemate") with phenylalanine methyl ester (referred to as "PM" hereinafter, wherein the term "PM" means "L-PM" or "PM racemate") in a water-soluble medium.

This invention also relates to a novel thermolysin-like protease which is highly stabilized.

Z-APM is a precursor to be used in the synthesis of α-L-aspartyl-L-phenylalanine methyl ester (referred to as "APM" hereinafter) which is an important artificial sweetener that has a degree of sweetness of about 200 times higher than that of sugar and has excellent taste properties such as no remaining bitterness.

### BACKGROUND ART

A method for the synthesis of benzyloxycarbonyl-α-L-aspartyl-L-phenylalanine methyl ester by an enzyme reaction making use of a thermolysin-like protease in the coupling reaction of N-benzyloxycarbonylaspartic acid with phenylalanine methyl ester in a water-soluble medium is described in Japanese Published Unexamined Patent No. 8-196292. According to this process, Z-Asp and L-PM are used on almost the same molar basis, pH of the reaction system is from 4.5 to 6.0, high concentration of salts is required and, though the activity of thermolysin in the reaction is fairly high, it is known that reduction of the enzyme activity at the time of the completion of the reaction is considerable in comparison with the activity at the time of the commencement of the reaction, so that such a reduction results in an increment of the amount of an enzyme to be used and therefore entails increased costs.

In this connection, in order to understand the present invention, the following shows information on thermolysin and its stability.

Thermolysin is a metalloprotein found for the first time in a culture medium of *Bacillus thermoproteolyticus* (Endo, S., *J. Fermentation Tech.*, 40, 346-353 (1962)). Thermolysin amino acid sequence (Titani, K. *et al, Nature New Biol.*, 238, 35-37 (1972); EP-A-0418625; Miki, Y., *J. Mol. Biol*., 160, 623-639 (1994)), three-dimensional structure (Vriend, G., *J. Computer-Aided Molecular Design*, 7, 367-396 (1993); Holmes, M.A. and Mattews, B.W., *J. Mol. Biol.*, 160, 623-639 (1982)) and catalytic mechanism (Kester, R.K. and Mattews, B.W., *J. Biol. Chem.*, 252, 7704-7710 (1982)) have been reported.

According to these reports, it was pointed out that the 143rd position glutamic acid residue and the 231st position histidine are important for the expression of protease activity in thermolysin, in addition to the zinc ion contained in the thermolysin molecule. Hereinafter, these 143rd and 231st position amino acid residues are referred to as " residues for the protease reaction".

A protease having similar amino acid sequence to that of thermolysin and similar catalytic activity to that of thermolysin has been reported in literature (for example, Imanaka *et al., Nature,* 324, 695-697 (1986)). Studies on the introduction of a mutation into a specified site of the amino acid sequence of thermolysin have also been reported in literature (Kubo, M. *et al., Appl. Environ. Microbiol.*, 58, 3779-3787 (1992)) and in a patent application (EP-A-0616033). In this patent application, it is shown that the introduction of mutations into the 144th, 150th, 187th and 227th positions improves the enzyme activity. Hereinafter, proteases having 1.2 to 10 times higher activity value than the Z-APM synthesizing activity value of wild type thermolysin are to be referred to as "thermolysin-like proteases".

Thus, the term "thermolysin-like protease" as used herein means a protease in which its amino acid sequence coincides with the amino acid sequence shown in the SEQ ID NO:1 of the Sequence Listing, or a protease in which one or more of the amino acid residues of the amino acid sequence shown in SEQ ID NO:1 of the Sequence Listing are replaced with other amino acid residues, or a metalloprotease in which one or more amino acid residues are inserted into one or more sites of the amino acid sequence or one or more amino acid residues are deleted therefrom.

The wild type thermolysin and the mutation-introduced thermolysin in such a manner are highly stable in a buffer solution of neutral range and show markedly high activity at such a pH. It is known that the stability of thermolysin and "thermolysin-like protease" is reduced within the weakly acidic range of about pH 4.5 to 6; and that thermolysin is completely inactivated at pH 4 or lower.

Also, it is known that the stability of thermolysin changes based on the concentration of benzyloxycarbonyl-α-aspartic acid (referred to as "Z-Asp" hereinafter) (Japanese Published Unexamined Patent No. 8-131170), and that patent application describes that thermolysin becomes stable when Z-Asp is allowed to be present at a molar ratio of 30 times or more, and that such a effect may be advantageous in carrying out a coupling reaction for the synthesis of Z-APM. In said patent, however, virtually nothing is described about the stability of thermolysin during the Z-APM synthesis which requires a prolonged period of time or about the Z-APM coupling formation reaction at a significantly low concentration of active catalyst.

In addition, a recent examination carried out by the inventors of the present invention has revealed that the stability of that enzyme is not sufficient within the pH range of 4.5 to 6 in this coupling reaction and that the coupling reaction cannot be carried out efficiently even at a Z-Asp concentration of 0.3 mole/liter or more.

On the other hand, according to the literature (Nakanishi *et al., Appl. Microbiol. Biotechnol.*, 32, 633-636 (1990)), it appears that the presence of Z-Asp rather exerts a bad influence upon the stability of thermolysin.

Contrary to thermolysin, Z-APM is not stable at a pH of about 7 but stable under a weakly acidic condition of pH 4.5 to 6.

The unstable nature of Z-APM at a pH of about 7 and the unstable nature of thermolysin at a pH of about 4.5 to 6 greatly impede efficient synthesis of Z-APM using thermolysin. When Z-APM is synthesized by a coupling reaction of Z-Asp with PM using thermolysin at a pH of about 7, thermolysin is stable but it causes a problem in that the recovery yield of Z-APM is reduced due to decomposition of the formed Z-APM and the starting material PM. Also, when this reaction is carried out at a pH of about 4.5 to 6, the activity of thermolysin is likely to decrease, so that the material and product are stable but efficient synthesis of Z-APM becomes difficult.

Consequently, improvement of the stability of thermolysin at a pH of about 4.5 to 6 will render possible efficient synthesis of Z-APM within this pH range.

A preceding literature (Japanese Published Unexamined Patent No. 6-181761) discloses that enzyme activities with respect to the Z-APM decomposition and Z-APM synthesis were improved when at least one amino acid residue among the 144th position leucine residue, the 150th position aspartic acid residue, the 187th position glutamic acid residue and the 227th position asparagine residue was replaced with another amino acid residue. In this case, the Z-Asp/PM molar ratio was about 0.5, and Z-APM was decomposed or synthesized at a pH of about 7. However, virtually nothing is examined on the stability of this protease, particularly on its stability when it is used in the synthesis of Z-APM at a Z-Asp/PM molar ratio of 0.8 to 1.7 at pH 7 or less.

Japanese Published Unexamined Patent No. 8-131170 described in the foregoing as another prior art reference teaches that the stability of a metalloprotease is improved by the addition of an N-protected amino acid. In this case, the Z-Asp/PM molar ratio was about 0.5, and Z-APM was decomposed or synthesized at a pH of about 6. However, also in this literature virtually nothing is examined on the stability of the protease, particularly on its stability when it is used in the synthesis of Z-APM at a Z-Asp/PM molar ratio of 0.8 to 1.7 at pH 6 or less.

Consequently, there was a necessity to find an improved process for lowering the reduction of the enzyme activity. In addition, more flexible reaction conditions are desirable with regard to the molar ratio of substrates and the pH range.

An object of the present invention is to solve the aforementioned problems, with the aim of improving the stability of the prior art thermolysin-like proteases, particularly their stability within the pH range of about 4.5 to 6.0, and thereby to provide means for improving methods in which benzyloxycarbonyl-α-L-aspartyl-L-phenylalanine methyl ester is synthesized by using a thus stabilized a thermolysin-like protease in the coupling reaction of Z-Asp with L-PM.

### DESCRIPTION OF THE INVENTION

The inventors of the present invention have analyzed the three-dimensional structure of thermolysin disclosed in literature (Vriend, G. and Eijsink, V., *J*. *Computer-Aided Molecular Design*, 7, 367-396 (1993); Holmes, M.A. and Mattews, B.W., *J. Mol. Biol*., 160, 623-639 (1982)) in detail and found as a result of their efforts that a cavity is present in the central part of the enzyme molecule and that it is possible to improve the stability of thermolysin-like protease if this cavity can be filled up. Thereafter, more in-depth examination has revealed that the volume of a cavity in electron density surrounded by the 144th position leucine cavity, the 149th position threonine residue, the 240th position alanine residue, the 270th position alanine residue and the 288th position alanine residue of the thermolysin-like protease can be reduced by filling up at least a portion of the cavity, by replacing one or more of these amino acid residues with other amino acid residues having higher hydrophobic character and being bulkier than that of the former amino acid residues, or through exertion of an influence upon the amino acid residues which surround the cavity in electron density by the replacement of peripheral amino acids thereof, and that the stability of such a type of thermolysin-like protease under conditions of low pH and high Z-Asp concentration is superior to that of other proteases, thus resulting in the accomplishment of the present invention.

Accordingly, the present invention relates to a method for the synthesis of Z-APM by an enzyme reaction in which a thermolysin-like protease is used for the coupling of Z-Asp with PM in a water-soluble medium, which comprises using a novel thermolysin-like protease in which the volume of an electron density cavity that exists in a thermolysin molecule having the amino acid sequence shown in SEQ ID NO:1 and is surrounded by at least the 144th position leucine residue, the 149th position threonine residue, the 240th position alanine residue, the 270th position alanine residue and the 288th position alanine residue, is reduced by filling up at least a portion of the cavity through replacement of one or more of these amino acid residues by other amino acid residues having higher hydrophobic character and being bulkier than those of the former amino acid residues, or through exertion of an influence upon the amino acid residues which surround the cavity in electron density, particularly using a novel thermolysin-like protease which is characterized in that the amino acid residues to be used in the replacement are preferably selected from the group consisting of histidine, isoleucine, leucine, phenylalanine, threonine, tyrosine and valine, more preferably in that the 144th position leucine residue is replaced with a phenylalanine residue or a tyrosine residue.

Table 1 shows the bulk size (unit: cm³/mol, see Zamyatnin, A.A., *Ann. Rev. Biophys. Bioeng.*, 13, 145-165 (1984)) and the degree of hydrophobic character (π value, see Fauchere, J.L. and Pliska, V., *Eur. J. Med. Chem.,* 18*,* 369-375 (1983)) of amino acid residues.

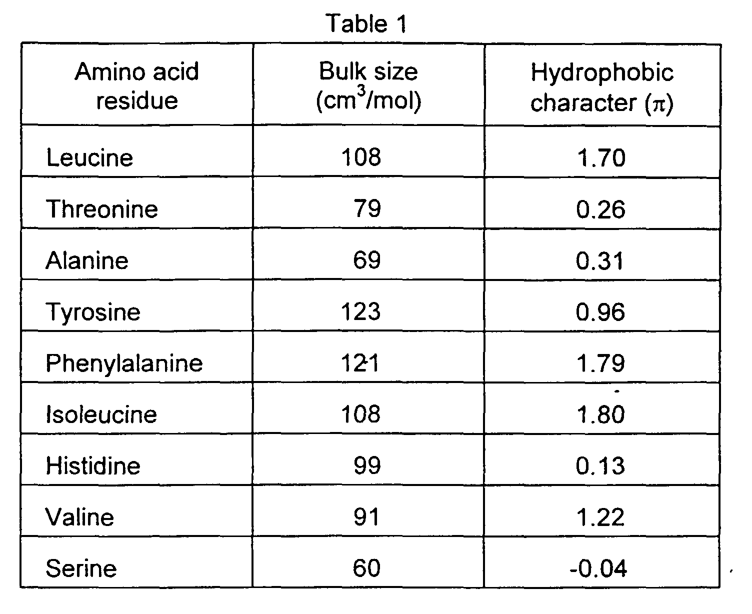

As is evident from this table, the replacement can be effected by selecting an amino acid residue being bulkier than that of the original amino acid residue, so that, for the 144th position leucine residue, for example, tyrosine residue and phenylalanine residue are suitable and phenylalanine residue is particularly desirable, because it is not only bulkier size but also has more hydrophobic character. For the 149th position threonine residue, tyrosine residue and phenylalanine residue, as well as isoleucine, histidine and valine, are suitable. Among these residues, those other than histidine are particularly desirable, because they are not only bulkier but also have more hydrophobic character.

The invention also relates to a method for use in the synthesis of Z-APM in a novel reaction system in which the prior art thermolysin-like proteases described in Japanese Published Unexamined Patent No. 6-181761 cannot be used, which is further characterized in that the pH of the water-soluble medium is from 4.3 to 8.0, the molar ratio of Z-Asp/PM is from 0.8 to 1.7 (it is generally 1:2) and the starting concentration of Z-Asp is 0.2 mol/kg or more, as the conditions for the synthesis of Z-APM.

With regard to methods for substituting amino acid residues, there is an M13 phage mutation formation method (Vandeyar, M. *et al., Gene*, 65, 129 (1988)) which is also described in Japanese Published Unexamined Patent No. 6-181761. According to this method, a recombinant phage M13TZBa-Sp is firstly prepared by cutting off a SphI-BamHI fragment from a plasmid pUCTZ55 and integrating the fragment into M13mp19 previously digested with restriction enzymes SphI and BamHI. Using the recombinant phage M13TZBa-Sp as a template, and a mutation introducing primer and a reverse direction primer, the region between both primers is amplified by PCR, and the thus obtained mutation-introduced DNA fragment is digested with restriction enzymes SphI and BamHI to substitute with the SphI-BamHI fragment of plasmid pUBTZ2 and then transformed into *Escherichia coli*, thereby obtaining a mutant.

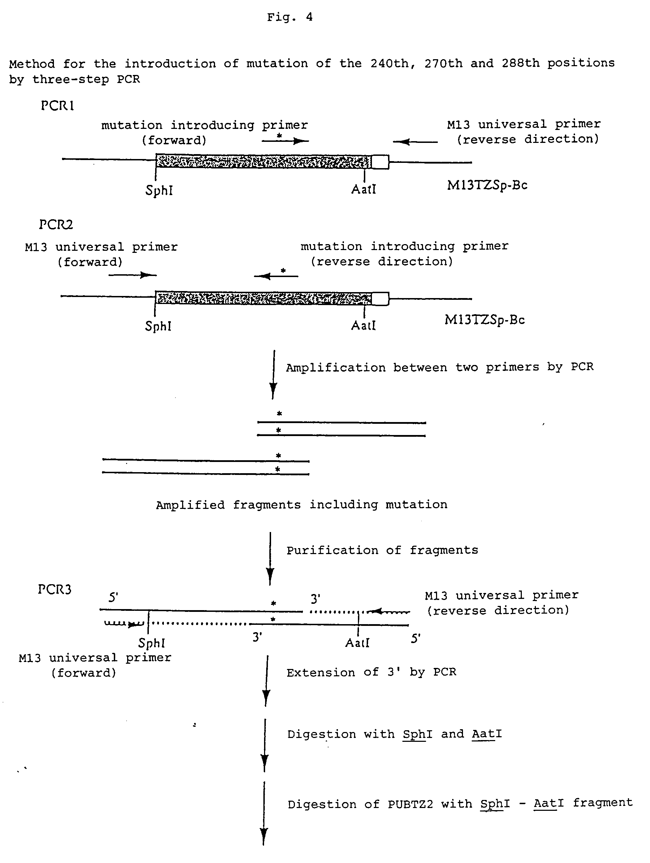

Using the recombinant phage M13TZBa-Sp as a template, and a forward mutation introducing primer and an M13 reverse direction mutation introducing primer, the region between both primers is amplified by PCR (PCR 1). Separately from this, using M13TZBa-Sp as a template, and a reverse direction mutation introducing primer and an M13 forward mutation introducing primer, the region between both primers is amplified by PCR (PCR 2). After removing primers from the reaction solution, the PCR is carried out using both mutation-introduced DNA fragments, the M13 forward mutation introducing primer and the M13 reverse direction mutation introducing primer, thereby effecting extension of both of the DNA fragments (PCR 3). The thus obtained mutation-introduced DNA fragments are digested with restriction enzymes SphI and BamHI to substitute with the SphI-BamHI fragment of plasmid pUBTZ2 and then transformed into *Escherichia coli*, thereby obtaining a mutant. This construction method is shown in Fig. 4.

### BRIEF DESCRIPTION OF THE DRAWINGS

Fig. 1 is a scheme showing steps for the construction of a plasmid pUCTZ55 from the plasmid pMK1.
Fig. 2 is a scheme showing steps for the construction of M13 phage M13TZBa-Sp from the plasmid pUCTZ55.
Fig. 3 is a scheme showing steps for the introduction of mutation into nprM gene cloned on the plasmid pUBTZ2 using M13TZBa-Sp.
Fig. 4 is a scheme showing a method for the introduction of mutation by PCR.
Fig. 5 is a graph showing results of the evaluation of stability of thermolysin-like proteases in which the 144th position leucine residue is replaced with other amino acid residues.

### Description of symbols:

S: serine, Q: glutamine, C: cysteine, W: tryptophan, E: glutamic acid, A: alanine, L: leucine, N: asparagine, H: histidine, T: threonine, F: phenylalanine, Y: tyrosine
Fig. 6 is a scheme showing steps for the construction of a plasmid (L114F-D150W-N227H) from a plasmid pUBTZ2(L114F) cloned with nprM gene in which the 144th position leucine residue is replaced with a phenylalanine residue and a plasmid pUBTZ2(D150W-N227H) cloned with a double mutation enzyme gene.
Fig. 7 is a graph showing results of the measurement of stability of thermolysin-like protease.

### BEST MODE OF CARRYING OUT THE INVENTION

Examples of the present invention and comparative examples are given below by way of illustration and not by way of limitation.

### Example 1

This example describes a method for the production of the title protease using the gene nprM of a protease having the same amino acid sequence of the aforementioned thermolysin. In this connection, the protease coded in the nprM gene is referred to as wild type enzyme hereinafter. That is, the following describes a method for the replacement of the 144th position leucine residue of the wild type enzyme with a phenylalanine residue or a tyrosine residue.

A 1 µg portion of plasmid pMK1 containing nprM was digested with BamHI and PstI, each in 5 units, at 37°C for 2 hours in 20 µl of a reaction solution (50 mM Tris-HCl buffer pH 7.5, 10 mM magnesium chloride, 100 mM sodium chloride and 1 mM DTT). A DNA fragment of about 3.7 kb was isolated from the resulting sample by 1% agarose gel electrophoresis and purified using Gene Clean DNA purification Kit manufactured by Bio 101 Inc.

On the other hand, 1 µg of plasmid vector pUC9 contained in 20 µl of a reaction solution (50 mM Tris-HCl buffer pH 7.5, 10 mM magnesium chloride, 100 mM sodium chloride and 1 mM DTT) was digested with PstI and BamHI, each in 5 units, at 37°C for 2 hours.

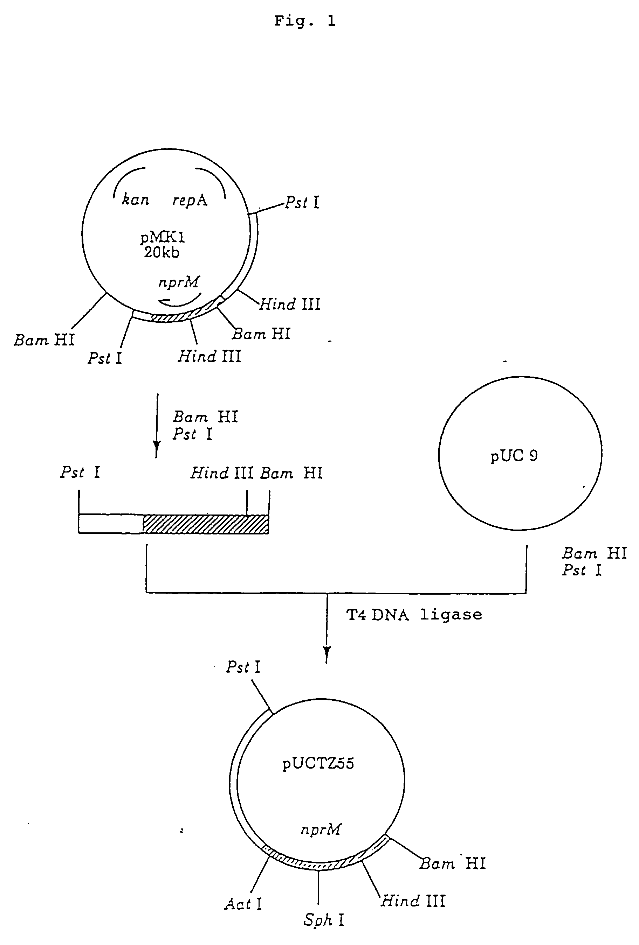

The thus obtained PstI-BamHI fragment of the nprM gene of pMK1 and the PstI-BamHI digest of pUC9 were allowed to react with each other using a DNA ligation kit manufactured by Takara Shuzo and then transformed into *Escherichia coli* JM109 in the usual way to obtain a recombinant plasmid (pUCTZ55) having the PstI-BamHI fragment of nprM gene (Fig. 1).

Thereafter, a 1 µg portion of the plasmid pUCTZ55 was digested with SphI and BamHI, each in 5 units, at 37°C for 2 hours in 20 µl of a reaction solution (50 mM Tris-HCl buffer pH 7.5, 10 mM magnesium chloride, 100 mM sodium chloride and 1 mM DTT). A DNA fragment of about 730 kb was isolated from the resulting sample by 1% agarose gel electrophoresis and purified using Gene Clean DNA purification Kit manufactured by Bio 101.

On the other hand, a 1 µg portion of phage vector M13mp19 was digested with SphI and BamHI, each in 5 units, at 37°C for 2 hours in 20 µl of a reaction solution (50 mM Tris-HCl buffer pH 7.5, 10 mM magnesium chloride, 100 mM sodium chloride and 1 mM DTT).

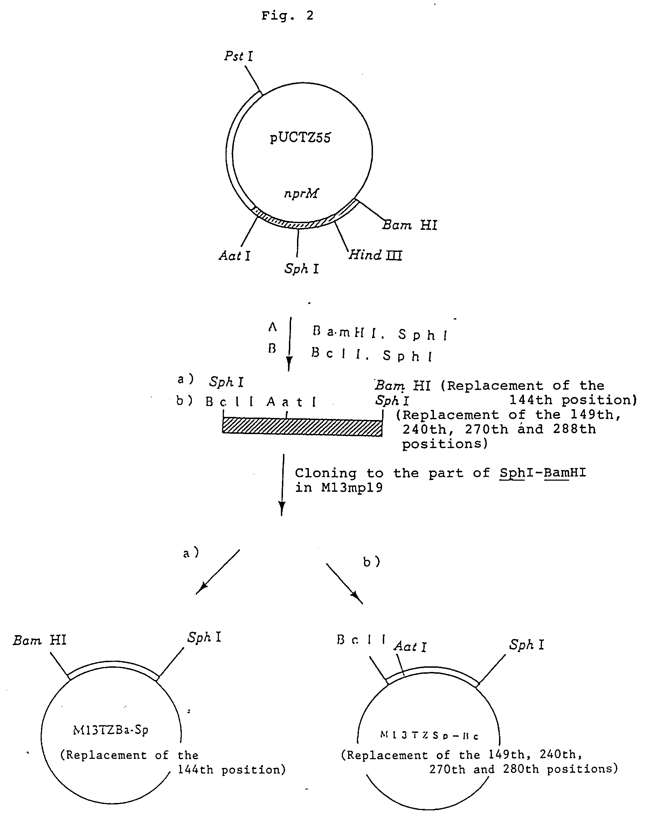

The thus obtained SphI-BamHI fragment of the nprM gene and the SphI-BamHI digest of M13mp19 were subjected to a ligation reaction using a DNA ligation kit manufactured by Takara Shuzo. By transforming the resulting reaction mixture into *Escherichia coli* JM109 in the usual way, a recombinant phage (M13TZBa-Sp) containing the SphI- BamHI fragment of nprM gene was obtained (Fig. 2).

A single-stranded DNA molecule was prepared from the thus obtained recombinant phage (M13TZBa-Sp) in the usual way and used in the introduction of amino acid replacement.

The oligonucleotide for amino acid replacement introduction, which was used in the replacement of the 144th position leucine residue by phenylalanine, is shown below and in the Sequence Listing as SEQ ID NO:2.

SEQ ID NO:2:

5'-TACCGCATGCGTGAACTCATGTG-3'

SphI Phe

Also, the other oligonucleotide for amino acid replacement introduction, which was used in the replacement by tyrosine, is shown below and in the Sequence Listing as SEQ ID NO:3.

SEQ ID NO:3:
5'-TACCGCATGCGTGTACTCATGTG-3'
SphI Tyr

These oligonucleotides were prepared using Model 380B DNA synthesizing apparatus manufactured by Applied Biosystems. They are referred to as reverse direction synthesis primers hereinafter.

A 0.5 µg portion of the thus prepared single-stranded DNA was dissolved in the PCR solution (67 mM Tris (pH 8.8), 16.6 mM ammonium sulfate, 6.7 mM magnesium chloride, 10 mM 2-mercaptoethanol, 0.2 mM dATP, 0.2 mM dGTP, 0.2 mM dTTP, 0.2 mM dCTP, 1 µM forward M13 universal primer and 1 µM reverse direction synthesis primer), and the resulting solution was mixed with 1 unit of Tth DNA polymerase, overlaid with 1 drop of mineral oil and then subjected to 30 cycles of the gene amplification reaction, each cycle including 1 minute of denaturation at 93°C, 1 minute of annealing at 45°C and 45 seconds of elongation at 72°C. After completion of the reaction, the water layer was recovered and extracted with phenol and ethanol precipitation in the usual way to recover the thus amplified DNA. Thereafter, BamHI and SphI, each in 5 units, were added to 20 µl of a reaction solution (50 mM Tris-HCl buffer pH 7.5, 10 mM magnesium chloride, 100 mM sodium chloride and 1 mM DTT) containing half the amount of the thus obtained DNA and allowed to react at 37°C for 2 hours. In this way, a fragment of mutation-introduced nprM gene having a size of about 730 bp was obtained.

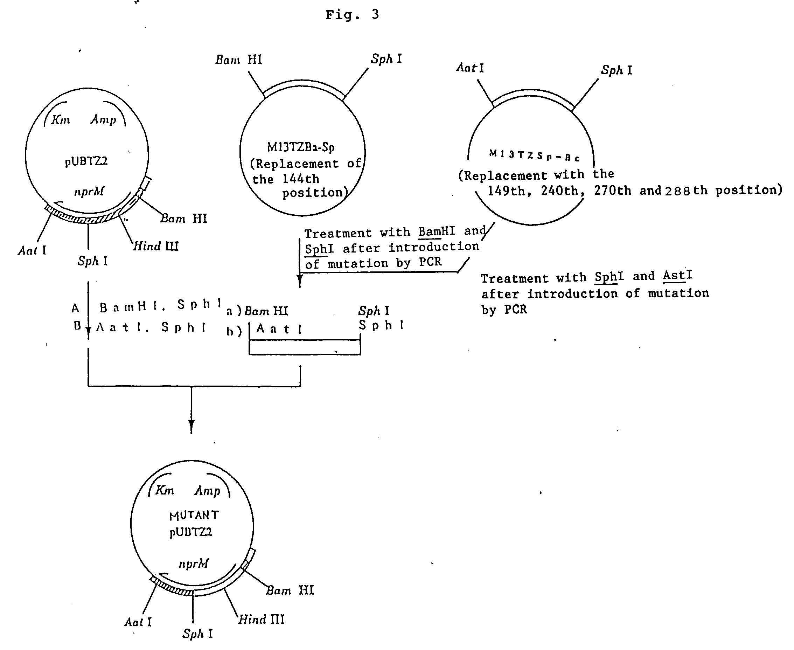

Separately from this, a shuttle vector already constructed by Miyake *et al*., namely a shuttle vector pUBTZ2 (Japanese Published Unexamined Patent No. 5-184363) which can be transformed into both *Escherichia coli* and *Bacillus subtilis* hosts and can express the nprM gene in both of these hosts, was treated with BamHI and SphI to obtain a fragment of 7.4 kb. This 7.4 kb fragment and the mutation-introduced 730 bp fragment were allowed to react with each other using a DNA ligation kit manufactured by Takara Shuzo and then transformed into *Escherichia coli* JM109 in the usual way, thereby obtaining recombinant plasmids pUBTZ2(L144F) and pUBTZ2(L144Y) in which the mutation had been introduced (Fig. 3).

A 3 ml portion of an alkali solution (0.2 N sodium hydroxide and 0.2% SDS) was added to an agar medium on which the transformed *Escherichia coli* had been grown, thereby effecting lysis of the cells, and 1 ml portion of the lysate was recovered. The thus recovered solution was mixed with 1 ml of 5 M potassium acetate, and the mixture was allowed to stand for 10 minutes in an ice bath and then centrifuged at 8,000 rpm for 20 minutes to remove the precipitate. The thus obtained supernatant was treated with phenol and ethanol precipitation in the usual way, and the resulting precipitate was dried under reduced pressure and then dissolved in 0.2 ml of TE (10 mM Tris-HCl buffer (pH 7.5) and 1 mM EDTA) to obtain a plasmid sample. *Bacillus subtilis* MT-2 was transformed with the thus prepared plasmid in the usual way and spread on an agar medium supplemented with 1% casein and 20 µg/ml of kanamycin, and then 140 halo-forming colonies were selected.

Each of these colonies was cultured overnight at 37°C in 3 ml of LB medium supplemented with 20 µg/ml of kanamycin, and the plasmid pUBTZ2 was extracted from the resulting culture medium in the usual way and treated with BamHI and SphI to recover a DNA fragment of 730 bp. Nucleotide sequence of each of the thus recovered DNA fragments was analyzed using DNA Sequencer 373A manufactured by Applied Biosystems (presently Perkin-Elmer), thereby selecting a colony having a gene nprM(L144F) in which the corresponding 144th position amino acid residue is a phenylalanine residue or a gene nprM(L144Y) in which it is a tyrosine residue.

The enzymes of interest L144F and L144Y were obtained in the following manner from the thus obtained strains having respective enzyme genes. That is, *Bacillus subtilis* MT-2/pUBTZ2(L144F) or *Bacillus subtilis* MT-2/pUBTZ2(L144Y) having the gene of the present invention was inoculated into 5 ml of LB medium supplemented with kanamycin to a concentration of 5 µg/ml and cultured at 37°C for 8 hours to be used as a seed culture. The thus obtained seed culture was inoculated into a 2L medium composed of two times larger amounts of the components of LB medium except for sodium chloride, and the strain was cultured at 37°C for 20 hours. Amount of the seed culture at the time of this culturing was adjusted such that absorbance of the culture medium at 660 nm became 0.02. After the culturing, the culture broth was centrifuged at 8,000 rpm for 20 minutes to obtain culture supernatant fluid, and ammonium sulfate was added to the supernatant to 60% saturation. When the thus obtained solution was allowed to stand at 4°C for 16 hours, said enzymes precipitated completely. The enzyme-containing precipitate was recovered by 20 minutes of centrifugation at 8,000 rpm. The thus recovered precipitate was dissolved in 100 ml of buffer A (10 mM Tris-HCl pH 9, 10 mM calcium chloride and 0.3 M ammonium sulfate), the resulting solution was passed through a column packed with 20 ml of Butyl Toyopearl to effect adsorption of the enzymes, and then buffer B (10 mM Tris-HCl pH 9 and 10 mM calcium chloride) was passed through the column at a flow rate of 1.5 ml/minute in order to elute the enzymes and impurities separately. The eluates containing said enzymes were recovered and subjected to salting out with 60% saturation ammonium sulfate, and the resulting precipitate was allowed to stand overnight at 4°C to effect aging. The resulting precipitate was centrifuged at 10,000 rpm for 10 minutes, the thus obtained precipitate was dissolved in 3 ml of buffer C (10 mM Tris-HCl pH 7.0, 10 mM calcium chloride and 100 mM sodium chloride) and then purified enzymes L144F and L144Y were obtained using a gel filtration column (TSK Gel G2000 SW 21.5 x 600 mm) and the just described buffer as the elution solution.

### Example 2

### (Evaluation of the stability of novel protease)

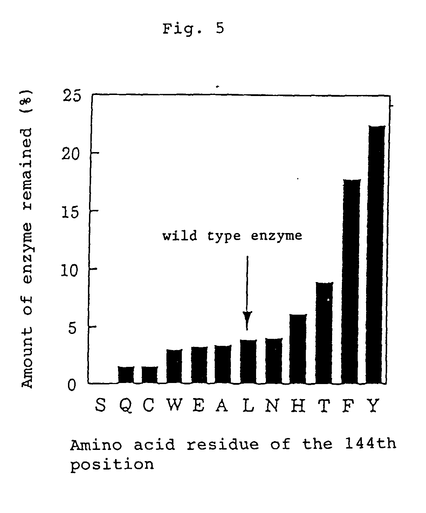

The purified L144F or L144Y was dissolved in 0.1 M sodium acetate buffer (pH 5.0) containing 0.8 M Z-Asp and 10 mM calcium chloride and kept at 40°C for 16 hours and then the amount of the enzyme remained in the solution was measured by a high performance liquid chromatography. TSK Gel Phenyl-5PW RP (4.6 mm I.D. x 7.5 cm) was used as the column of the high performance liquid chromatography, and the elution was carried out using 10 mM potassium acetate containing acetonitrile as the elution solution at a flow rate of 1.2 ml/min. Detection of the enzyme was carried out by the optical absorption at 280 nm. Residual ratio of the enzyme obtained by the culturing and contained in the solution is shown in Fig. 5. Both of the L144Y enzyme and L144F enzyme showed about two times higher residual ratio than that of the wild type enzyme having the same sequence as the thermolysin-like amino acid sequence, thereby revealing that both of the enzymes of the present invention are having high stability.

### Comparative Example 1

Thermolysin-like proteases in which the 144th position leucine residue was replaced with an amino acid residue other than phenylalanine residue and tyrosine residue were produced and their stability was examined in comparison with Example 2. These proteases were produced by the same procedure of Example 1, except that a random primer having a nucleotide sequence of the SEQ ID NO:4 shown below and in the Sequence Listing was used, and examination of the stability of these proteases was carried out by the same procedure of Example 2.

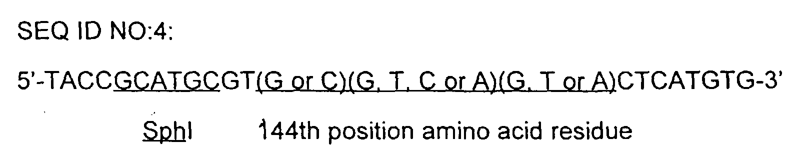

The proteases in which the 144th position leucine residue was replaced with an amino acid residue other than a phenylalanine residue and a tyrosine residue were identified by the determination of plasmid nucleotide sequences.

Results of the examination of their stability are shown in Fig. 4. It was found that the stability of both L144T (leucine residue was replaced with threonine residue) and L144H (leucine residue was replaced with histidine residue) is higher than that of the wild type enzyme but lower than the stability of L144F or L144Y. Stability of L144S was lower than that of the wild type enzyme.

### Example 3

A double mutation protease (D150W-N227H in which the 150th position aspartic acid residue was replaced with a tryptophan residue and the 227th position asparagine residue was replaced with a histidine residue) having high activities for Z-APM decomposition and Z-APM synthesis was used as the starting material for the production of a high stability enzyme.

Plasmid was extracted from *Bacillus subtilis* transformant MT-2/pUBTZ2(D150W-N227H) in the usual way, and 1 µg portion of the plasmid contained in 20 µl of a reaction solution (50 mM Tris-HCl buffer pH 7.5, 10 mM magnesium chloride, 100 mM sodium chloride and 1 mM DTT) was digested with 5 units each of AatI and SphI at 37°C for 2 hours and then allowed to undergo 5 minutes of the reaction at 70°C. By subjecting the thus prepared sample to 1% agarose gel electrophoresis, a DNA fragment of about 530 bp corresponding to the D150W-N227H having double mutation was obtained.

Separately from this, 1 µg of the plasmid pUBTZ2 containing the mutation of L144F prepared in Example 1 was digested with 5 units each of AatI and SphI at 37°C for 2 hours and then allowed to undergo 5 minutes of the reaction at 70°C. By subjecting the thus prepared sample to 1% agarose gel electrophoresis, a DNA fragment of about 7.6 kb containing the mutation of L144F was obtained.

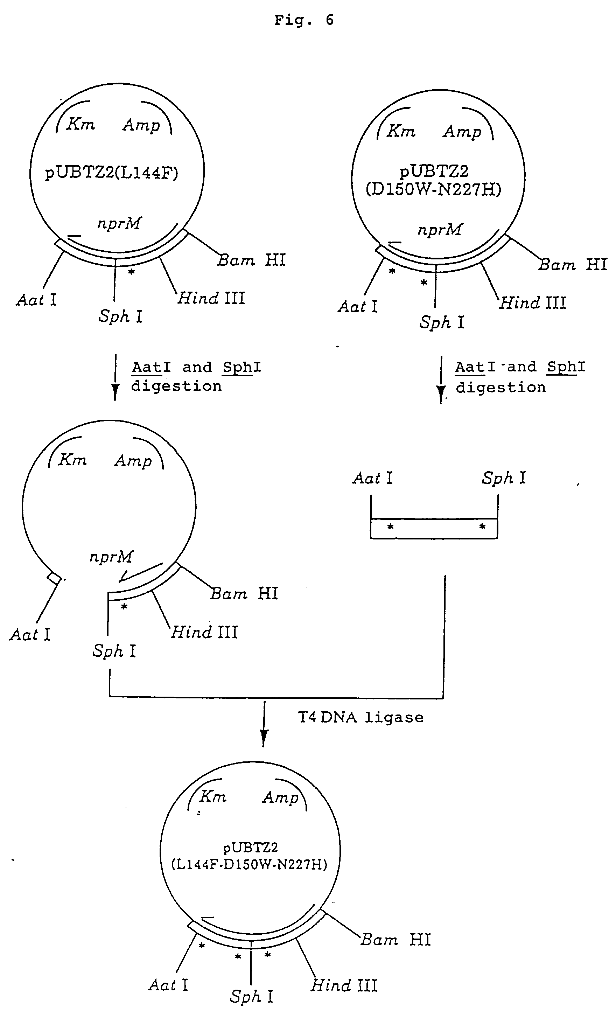

The DNA fragment of about 7.6 kb obtained from the plasmid pUBTZ2(L144F) in this manner and the DNA fragment of about 530 bp obtained from the plasmid pUBTZ2(D150W-N227H) in this manner were linked to each other using a ligation kit manufactured by Takara Shuzo, and the reaction mixture was transformed into *Escherichia coli* JM103 in the usual way to obtain a transformant JM103/pUBTZ2 in which the mutations had been introduced. The replaced amino acid residues were confirmed by identification of the plasmid nucleotide sequence. The method for the construction of the recombinant plasmid pUBTZ2(L144F-D150W-N227H) is shown in Fig. 6.

Using this plasmid DNA, *Bacillus subtilis* transformant MT-2/pUBTZ2(L144F-D150W-N227H) was prepared in the same manner as in Example 1, and *Bacillus subtilis* transformant MT-2/pUBTZ2(L144Y-D150W-N227H) was also prepared.

Purification of the two mutant enzymes L144F-D150W-N227H and L144Y-D150W-N227H from these *Bacillus subtilis* transformants was carried out in accordance with the method shown in Example 1, except that a buffer solution D (10 mM Tris-HCl pH 8.5, 0.5 M guanidine hydrochloride and 10 mM calcium chloride) was used in stead of the buffer solution B.

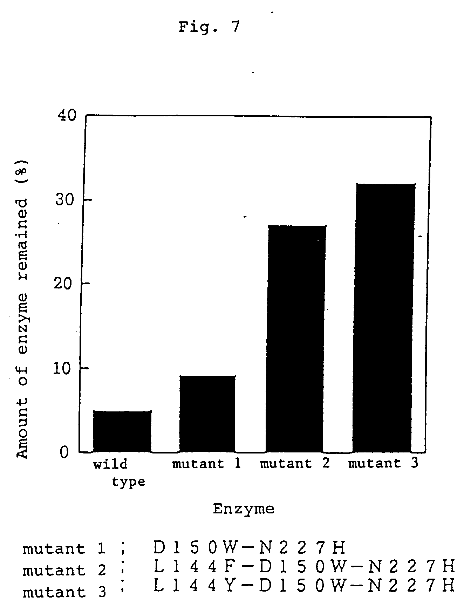

When the stability of the thus prepared enzymes was evaluated in the same manner as in Example 2, both of the L144F-D150W-N227H and L144Y-D150W-N227H showed higher stability in comparison with the wild type enzyme and the original D150W-N227H. Results of the stability evaluation are shown in Fig. 7.

### Example 4

Practical synthesis of Z-APM was carried out using the L144F-D150W-N227H prepared by the method of Example 3. A suspension was prepared by dissolving L-PM (189 g; 712.6 mmol) and Z-Asp (113 g; 633.3 mmol) in water. The solution was adjusted to pH 5.3 with 22% sodium hydroxide solution, and 155 g of sodium chloride was added thereto. The thus prepared solution was clear. At the same time, an enzyme solution was prepared by adding 2.5 g of the enzyme and 3.36 g of CaCl2 to water.

Both of these solutions were mixed and an enzyme coupling reaction was started. The solution after mixing has a composition shown in the following Table 2.

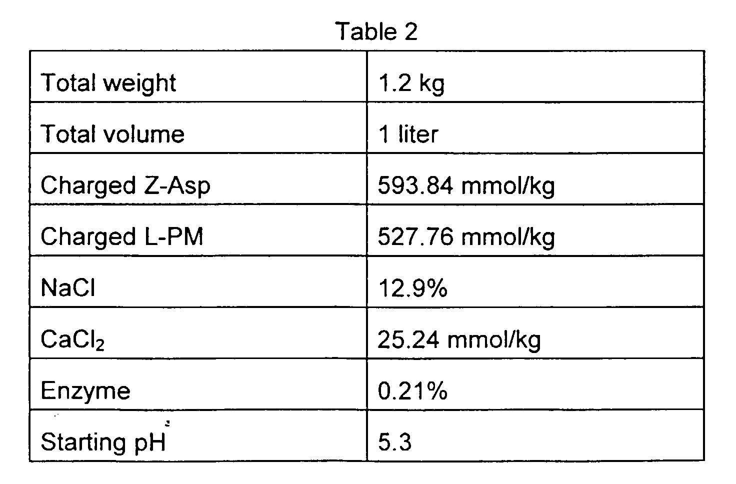

The enzyme reaction was carried out in a glass reaction vessel having a diameter of about 13 cm which was warmed at 40°C. On a position 0.5 cm from the bottom of this reaction vessel was arranged a variable speed agitator having a blade of 11 cm in length set at a level of depth 2 cm from the liquid surface. The agitation speed was controlled at 150 revolutions per minute for the first 50 minutes after commencement of the reaction and then at 300 revolutions per minute thereafter. The pH of the reaction mixture was measured continuously during the reaction and adjusted to 5.3 by adding 16% hydrochloric acid when the pH increased to 5.3 or more. After a predetermined period of time, a sample was taken out from the reaction vessel to analyze concentrations of Z-Asp, L-PM and residual enzyme by the following HPLC operation.

Z-Asp and L-PM were measured quantitatively by an HPLC system in which a G2000SW XL (TSK-GEL) column was used, at 25°C and at a flow rate of 1 ml/min. A 70/30 acetonitrile/water mixture solution containing 0.15 M acetate buffer of pH = 5.6 was used as the elution solution. The eluted components were detected at 254 nm.

Also, the concentration of the remaining enzyme was measured quantitatively by an HPLC system in which a Phenyl-5PW RP (TSK-GEL) column was used, at 25°C and at a flow rate of 1.2 ml/min. In this analysis, a gradient elution solution consisting of an eluent A (a 5/95 acetonitrile/water mixed solution containing a calcium acetate buffer of pH = 6) and an eluent B (a 60/40 acetonitrile/water mixed solution containing a calcium acetate buffer of pH = 6) was used. The gradient was effected by: keeping A/B = 90/10 for the first 0 to 4 minutes and then changing A/B = from 90/10 to 40/60 for the remaining 6 minutes of 4 to 10 minutes. The eluted enzyme was detected at the absorbance at 280 nm.

The relationship between the conversion ratio of Z-APM from Z-Asp and the residual ratio of the enzyme is shown in Table 3.

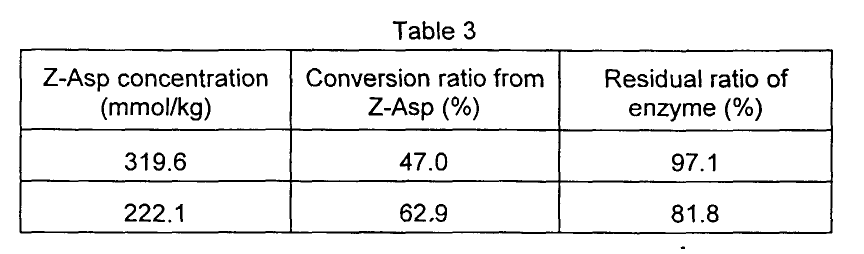

### Comparative Examples 2 and 3

The procedure of Example 4 was repeated using wild type enzyme and the D150W-N227H obtained in Example 3 and using the solutions shown in Table 4 having slightly different compositions.

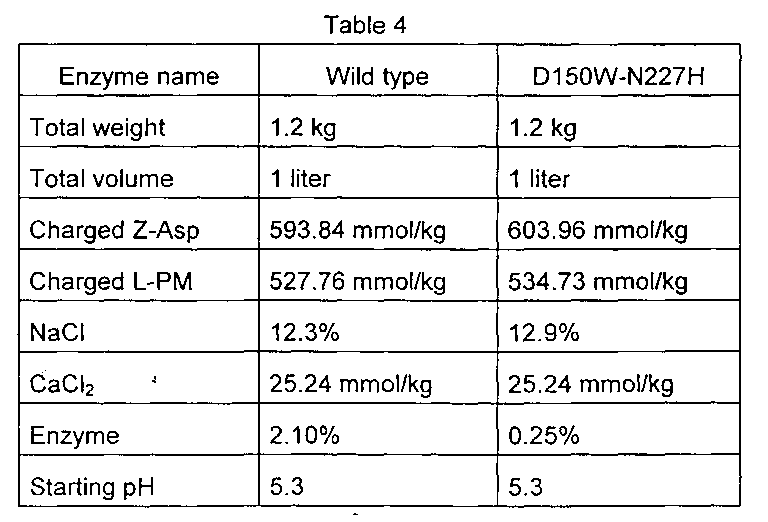

The results obtained are shown in the following Table 5.

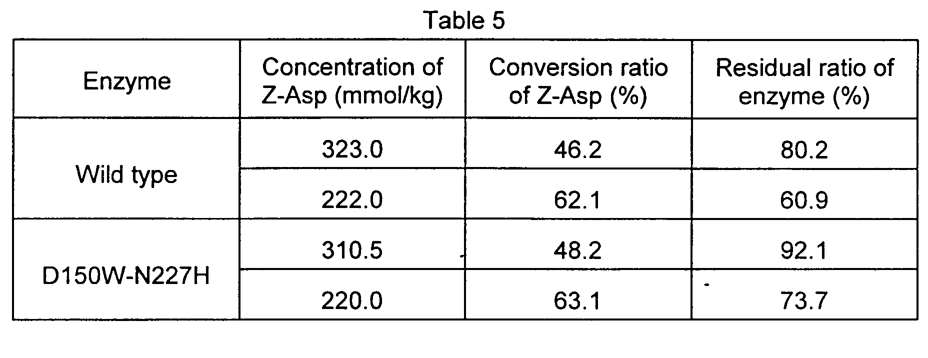

As is evident from the above table, the triple mutation enzyme (L144F-D150W-N227H) of the present invention has excellent stability in comparison with the prior art wild type enzyme and the double mutation enzyme (D150W-N227H).

### INDUSTRIAL APPLICABILITY

The novel thermolysin-like protease of the present invention has excellent stability in comparison with the wild type thermolysin and other mutation-introduced thermolysin-like proteases (for example, a thermolysin-like protease in which the 150th position aspartic acid residue is replaced with tryptophan residue and the 227th position aspartic acid residue is replaced with histidine residue), and, particularly, it is stable even in a solution having a pH of about 5. In addition, this thermolysin-like protease can be used even in a new Z-APM synthesis system which cannot be effected by the prior art thermolysin-like protease, a system in which a molar ratio of the starting materials Z-Asp and L-PM is 0.8 to 1.7 (generally 2) and pH of the reaction solution is 4.3 to 3.8 (generally 7). Because of these advantages, excellent effects can be obtained in terms, for example, of the continuation of the reaction for a prolonged period of time and improvement of the enzyme recovery yield after the reaction.

In this connection, the Sequence Listing according to the present invention is as shown in the following.

SEQUENCE LISTING

SEQ ID NO:1
SEQUENCE LENGTH: 316
SEQUENCE TYPE: amino acid
TOPOLOGY: linear
MOLECULE: protein
ORIGIN: the genus Bacillus
SEQUENCE DESCRIPTION:
Ile Thr Gly Thr Ser Thr Val Gly Val Gly Arg Gly Val Leu Gly
1               5                   10                  15
Asp Gln Lys Asn Ile Asn Thr Thr Tyr Ser Thr Tyr Tyr Tyr Leu
                20                  25                  30
Gln Asp Asn Thr Arg Gly Asn Gly Ile Phe Thr Tyr Asp Ala Lys
                35                  40                  45
Tyr Arg Thr Thr Leu Pro Gly Ser Leu Trp Ala Asp Ala Asp Asn
                50                  55                  60
Gln Phe Phe Ala Ser Tyr Asp Ala Pro Ala Val Asp Ala His Tyr
                65                  70                  75
Tyr Ala Gly Val Thr Tyr Asp Tyr Tyr Lys Asn Val His Asn Arg
                80                  85                  90
Leu Ser Tyr Asp Gly Asn Asn Ala Ala Ile Arg Ser Ser Val His
                95                  100                 105
Tyr Ser Gln Gly Tyr Asn Asn Ala Phe Trp Asn Gly Ser Gln Met
                110                 115                 120
Val Tyr Gly Asp Gly Asp Gly Gln Thr Phe Ile Pro Leu Ser Gly
                125                 130                 135
Gly Ile Asp Val Val Ala His Glu Leu Thr His Ala Val Thr Asp
                140                 145                 150
Tyr Thr Ala Gly Leu Ile Tyr Gln Asn Glu Ser Gly Ala Ile Asn
                155                 160                 165
Glu Ala Ile Ser Asp Ile Phe Gly Thr Leu Val Glu Phe Tyr Ala
                170                 175                 180
Asn Lys Asn Pro Asp Trp Glu Ile Gly Glu Asp Val Tyr Thr Pro
                185                 190                 195
Gly Ile Ser Gly Asp Ser Leu Arg Ser Met Ser Asp Pro Ala Lys
                200                 205                 210
Tyr Gly Asp Pro Asp His Tyr Ser Lys Arg Tyr Thr Gly Thr Gln
                215                 220                 225
Asp Asn Gly Gly Val His Ile Asn Ser Gly Ile Ile Asn Lys Ala
                230                 235                 240
Ala Tyr Leu Ile Ser Gln Gly Gly Thr His Tyr Gly Val Ser Val
                245                 250                 255
Val Gly Ile Gly Arg Asp Lys Leu Gly Lys Ile Phe Tyr Arg Ala
                260                 265                 270
Leu Thr Gln Tyr Leu Thr Pro Thr Ser Asn Phe Ser Gln Leu Arg
                275                 280                 285
Ala Ala Ala Val Gln Ser Ala Thr Asp Leu Tyr Gly Ser Thr Ser

                290                 295                 300
Gln Glu Val Ala Ser Val Lys Gln Ala Phe Asp Ala Val Gly Val
                305                 310                 315
Lys

SEQ ID NO:2
SEQUENCE LENGTH: 23
SEQUENCE TYPE: nucleic acid
TOPOLOGY: linear
MOLECULE: other nucleic acid (synthetic DNA)
SEQUENCE DESCRIPTION:
TACCGCATGC GTGAACTCAT GTG 23

SEQ ID NO:3
SEQUENCE LENGTH: 23
SEQUENCE TYPE: nucleic acid
TOPOLOGY: linear
MOLECULE: other nucleic acid (synthetic DNA)
SEQUENCE DESCRIPTION:
TACCGCATGC GTGTACTCAT GTG 23

SEQ ID NO:4
SEQUENCE LENGTH: 23
SEQUENCE TYPE: nucleic acid
TOPOLOGY: linear
MOLECULE: other nucleic acid (synthetic DNA)
SEQUENCE DESCRIPTION:
TACCGCATGC GTSNDCTCAT GTG 23

## Claims

1. A method for synthesizing benzyloxycarbonyl-α-L-aspartyl-L-phenylalanine methyl ester by an enzyme reaction in which a thermolysin-like protease is used for the coupling of N-benzyloxycarbonylaspartic acid with phenylalanine methyl ester in a water-soluble medium, which comprises using a novel thermolysin-like protease in which the volume of an electron density cavity that exists in a thermolysin molecule having the amino acid sequence shown in the SEQ ID NO:1 of the Sequence Listing and is surrounded by at least the 144th position leucine residue, the 149th position threonine residue, the 240th position alanine residue, the 270th position alanine residue and the 288th position alanine residue, is reduced by filling up at least a portion of the cavity through replacement of one or more of these amino acid residues by other amino acid residues having higher hydrophobic character and bulkier than those of the former amino acid residues, or through exertion of an influence upon the amino acid residues which surround the cavity in electron density.

2. The method for synthesizing benzyloxycarbonyl-α-L-aspartyl-L-phenylalanine methyl ester by an enzyme reaction according to claim 1, wherein the volume of the electron density cavity is reduced through the replacement of the 144th position, the 149th position, the 240th position, the 270th position and the 288th position amino acid residues by other amino acid residues selected from the group consisting of histidine, isoleucine, leucine, phenylalanine, threonine, tyrosine and valine which have larger molar volumes than those of the former residues.

3. The method for synthesizing benzyloxycarbonyl-α-L-aspartyl-L-phenylalanine methyl ester by an enzyme reaction according to claim 2, wherein the 144th position leucine residue is replaced with a phenylalanine residue or a tyrosine residue.

4. The method for synthesizing benzyloxycarbonyl-α-L-aspartyl-L-phenylalanine methyl ester by an enzyme reaction according to any one of claims 1 to 3, wherein at least one of the 150th position aspartic acid residue, the 187th position glutamic acid residue and the 227th position asparagine residue is replaced with another amino acid residue.

5. The method for synthesizing benzyloxycarbonyl-α-L-aspartyl-L-phenylalanine methyl ester by an enzyme reaction according to any one of claims 1 to 4, wherein the enzyme coupling reaction is carried out at a pH of 4.3 to 8.0.

6. The method for synthesizing benzyloxycarbonyl-α-L-aspartyl-L-phenylalanine methyl ester by an enzyme reaction according to claim 5, wherein N-benzyloxycarbonyl-L-aspartic acid and L-phenylalanine methyl ester are used as substrates of the enzyme coupling.

7. The method for synthesizing benzyloxycarbonyl-α-L-aspartyl-L-phenylalanine methyl ester by an enzyme reaction according to claim 6, wherein the molar ratio of N-benzyloxycarbonyl-L-aspartic acid to L-aspartyl-L-phenylalanine methyl ester is from 0.8 to 1.7.

8. The method for synthesizing benzyloxycarbonyl-α-L-aspartyl-L-phenylalanine methyl ester by an enzyme reaction according to any one of claims 1 to 7, wherein the concentration of N-benzyloxycarbonyl-L-aspartic acid is 0.2 mol/kg or more.

9. A novel thermolysin-like protease in which the volume of an electron density cavity that exists in a thermolysin molecule having the amino acid sequence shown in the SEQ ID NO:1 of the Sequence Listing and is surrounded by at least the 144th position leucine residue, the 149th position threonine residue, the 240th position alanine residue, the 270th position alanine residue and the 288th position alanine residue, is reduced by filling up at least a portion of the cavity through replacement of one or more of these amino acid residues by other amino acid residues having higher hydrophobic character and being bulkier than those of the former amino acid residues, or through exertion of an influence upon the amino acid residues which surround the cavity in electron density.

10. The novel thermolysin-like protease according to claim 9, wherein the volume of the electron density cavity is reduced through the replacement of at least one of the 144th position leucine residue, the 149th position threonine residue, the 240th position alanine residue, the 270th position alanine residue and the 288th position alanine residue by other amino acid residues selected from the group consisting of histidine, isoleucine, leucine, phenylalanine, threonine, tyrosine and valine which have larger molar volumes than those of the former residues.

11. The novel thermolysin-like protease according to claim 9, wherein the 144th position leucine residue is replaced with a tyrosine residue.

12. The novel thermolysin-like protease according to claim 9, wherein at least one of the 150th position aspartic acid residue, the 187th position glutamic acid residue and the 227th position asparagine residue is replaced with another amino acid.

13. A method for synthesizing benzyloxycarbonyl-α-L-aspartyl-L-phenylalanine methyl ester by an enzyme reaction and a highly stabilized thermolysin-like protease, as are substantially described in the specification and examples.
